# EUROPÄISCHE PATENTSCHRIFT

(11) **EP 2 691 364 B1**
(45) Veröffentlichungstag und Bekanntmachung des Hinweises auf die Patenterteilung: **04.03.2015**
(21) Anmeldenummer: 12710512.0
(22) Anmeldetag: 26.03.2012
(51) Int. Cl.: C07C 263/10, C01B 7/04, C07C 265/00

(54) **VERFAHREN ZUR HERSTELLUNG VON ISOCYANATEN**
METHOD FOR PRODUCING ISOCYANATES
PROCÉDÉ DE PRODUCTION D'ISOCYANATES

(30) Priorität: 31.03.2011 EP 11160575
(43) Veröffentlichungstag der Anmeldung: 05.02.2014
(73) Patentinhaber: BASF SE, 67056 Ludwigshafen (DE)
(72) Erfinder: LEHR, Vanessa, Simone, 68199 Mannheim (DE); MATTKE, Torsten, 67251 Freinsheim (DE); KNÖSCHE, Carsten, 67150 Niederkirchen (DE); SCHELLING, Heiner, 67281 Kirchheim (DE); OLBERT, Gerhard, 69221 Dossenheim (DE)
(86) Internationale Anmeldenummer: PCT/EP2012/055294
(87) Internationale Veröffentlichungsnummer: WO 2012/130788

(56) Entgegenhaltungen:
- WO-A1-2010/060773
- DE-A1-102004 041 777
- DE-A1-102006 024 549
- DE-A1-102007 020 096
- US-A1- 2009 149 671

## Beschreibung

Die Erfindung betrifft ein Verfahren zur Herstellung von Isocyanaten durch Umsetzung der entsprechenden Amine mit Phosgen, gegebenenfalls in Gegenwart eines Inertmediums, in der Gasphase, sowie eine Verwendung des Koppelproduktes Chlorwasserstoff der Gasphasenphosgenierung.

Die Gasphasenphosgenierung zur Herstellung von Isocyanaten hat gegenüber der Flüssigphasenphosgenierung die Vorteile, dass eine höhere Selektivität, ein geringerer Hold-Up an toxischem Phosgen sowie ein verminderter Energiebedarf und niedrigere Investitionskosten erforderlich sind.

Bei der Gasphasenphosgenierung wird ein aminhaltiger Eduktstrom mit einem phosgenhaltigen Eduktstrom jeweils in gasförmigem Zustand vermischt und die entsprechenden Diisocyanate unter Freisetzung von Chlorwasserstoff gebildet. Die Reaktanden müssen zuvor verdampft und auf Temperaturen über 300 °C überhitzt werden. Diese Verdampfung/Überhitzung erfolgt im Wesentlichen indirekt über elektrische Beheizung, über Verbrennungsgase oder über einen hochgespannten Dampf. Eventuell kann auch ein sekundäres Wärmeträgermedium dazwischengeschaltet werden. Die Bereitstellung der Energie, insbesondere zur Verdampfung, ist sehr kostenintensiv.

Die Synthese des in der Phosgenierung der Amine zur Herstellung von Isocyanaten eingesetzten Phosgens erfolgt im Allgemeinen unmittelbar der Phosgenierung vorgeschaltet. Hierbei wird Kohlenmonoxid mit Chlor unter Freisetzung von Wärme (Reaktionsenthalpie 110 kJ/mol), die abgeführt werden muss, da oberhalb von 100 °C ein vollständiger Umsatz wegen der Lage des Reaktionsgleichgewichtes nicht mehr möglich ist, zu Phosgen umgesetzt.

In der Gasphasenreaktion zur Synthese von Phosgen wird Kohlenmonoxid in der Regel im Überschuss eingesetzt, um ein Durchschlagen des Chlors zu verhindern, d.h. um weitgehend chlorfreies Phosgen zu erhalten.

Die DE-A1 10 2009 032 413 beschreibt ein Verfahren zur Gasphasenphosgenierung mit einer energetisch und apparativ vorteilhaften Gewinnung und Rückführung des Phosgens in die Phosgenierreaktion, das gleichzeitig eine erhöhte Sicherheit gewährleistet: primäre Armine werden mit Phosgen im stöchiometrischem Überschuss in der Gasphase umgesetzt, wobei
a) ein das Isocyanat enthaltender flüssiger Strom und ein Gasstrom enthaltend Chlorwasserstoff und Phosgen erhalten wird, wobei mit
b) der in Schritt a) erhaltene Gasstrom enthaltend Chlorwasserstoff und Phosgen zunächst in einen Chlorwasserstoff enthaltenden Gasstrom und einen Phosgen enthaltenden flüssigen Strom aufgetrennt wird, in Schritt
c) der in Schritt b) erhaltene Phosgen enthaltende flüssige Strom zumindest teilweise in einen gasförmigen Strom enthaltend Phosgen überführt wird, und
d) der in Schritt c) erhaltene gasförmige Strom enthaltend Phosgen in die Phosgenierungsreaktion in Schritt a) rückgeführt wird, wobei
e) der Druck des in Schritt c) erhaltenen gasförmigen Stroms enthaltend Phosgen höher ist als der Druck des in Schritt b) erhaltenen Phosgen enthaltenden flüssigen Stroms.

Das für die Phosgenierung notwendige Frisch-Phosgen kann zunächst verflüssigt und dadurch von inerten Gasen und Nebenprodukten der Phosgenherstellung weitestgehend gereinigt und anschließend verdampft werden.

Bei der Phosgenierung von Aminen zu Isocyanaten fällt ein chlorwasserstoffhaltiges Abgas als Koppelprodukt an, das in der Regel einer weiteren Verwendung zugeführt wird, wobei noch darin enthaltene Reste an Kohlenmonoxid nachteilige Wirkungen haben können.

Daher schlägt die DE-A1 10 2006 024 549 ein gekoppeltes Verfahren zur Synthese von organischen Isocyanaten vor, das die Herstellung von Phosgen durch Umsetzung von Kohlenmonoxid mit Chlor, die Umsetzung des Phosgens mit organischen Aminen unter Bildung der entsprechenden Isocyanate und die Abtrennung der organischen Isocyanate umfasst, und wobei das Kohlenmonoxid aus dem chlorwasserstoffhaltigen Abgas der Isocyanatsynthese durch Umsetzung mit Chlor unter Bildung von Phosgen entfernt wird. Das Phosgen wird abgetrennt und kann gegebenenfalls in eine Isocyanatsynthese recycliert werden. Das chlorwasserstoffhaltige, Kohlenmonoxid-verarmte Gas wird vorzugsweise einer Chlorwasserstoffoxidation (Deacon-Prozess) unterworfen. Das Verfahren ermöglicht es somit, Kohlenmonoxid aus den chlorwasserstoffhaltigen Abgasen der Isocyanatsynthese abzutrennen, um dadurch verursachte Nachteile, insbesondere in einem sich anschließenden Deacon-Prozess zu vermeiden, und gleichzeitig das Kohlenmonoxid einer möglichst wirtschaftlichen Verwendung zuzuführen.

Das Dokument weist darauf hin, dass in Gasphasenphosgenierungen Kohlenmonoxidgehalte bis über 5 % erreicht werden können, weil in derartigen Verfahren in der Regel keine Kondensation des Phosgens und somit keine Abtrennung des nicht reagierten Kohlenmonoxids vor der Phosgenierung erfolgt.

Demgegenüber wurde jedoch gefunden, dass das Kohlenmonoxid, das im phosgenhaltigen Einsatzstrom enthalten ist, sofern dieser unmittelbar aus der Synthese von Kohlenmonoxid und Chlor kommt, die mit einem stöchiometrischen Kohlenmonoxidüberschuss gefahren werden muss, in der Gasphasenphosgenierung zu Problemen führt: Bei Temperaturen oberhalb von 300 °C, liegt das Boudouard-Gleichgewicht, wonach Kohlenmonoxid in Kohlenstoff und Kohlendioxid disproportioniert, fast vollständig auf der Seite des CO₂. Der gebildete Kohlenstoff führt zu Apparate-Fouling und kann darüber hinaus die Zersetzung von Phosgen zu Chlor und Kohlenmonoxid katalysieren, welches sich wiederum nach dem Boudouard-Gleichgewicht unter Bildung von weiterem Kohlenstoff zersetzt, der wiederum die Zersetzung von weiterem Phosgen katalysiert.

Es war daher Aufgabe der Erfindung, ein Verfahren zur Herstellung von Isocyanaten durch Phosgenierung der entsprechenden Amine in der Gasphase zur Verfügung zu stellen, das die obigen Nachteile nicht aufweist.

Die Lösung besteht in einem Verfahren zur Herstellung von Isocyanaten durch Phosgenierung der entsprechenden Amine in der Gasphase, das dadurch gekennzeichnet ist, dass
- ein phosgenhaltiges Reaktionsgemisch aus der Gasphasensynthese von Kohlenmonoxid und Chlor zu Phosgen, die mit einem stöchiometrischen KohlenmonoxidÜberschuss gegenüber Chlor gefahren wird,
- mittels eines thermischen Trennverfahrens und/oder eines Membrantrennverfahrens in zwei Ströme aufgeteilt wird, und zwar in einen ersten, kohlenmonoxidarmen Strom, enthaltend maximal 1 Gew.-% Kohlenmonoxid, bezogen auf das Gesamtgewicht des ersten, kohlenmonoxidarmen Stromes, sowie in
- einen zweiten, kohlenmonoxidreichen Strom, enthaltend mehr als 10 Gew.% Kohlenmonoxid, bezogen auf das Gesamtgewicht des zweiten, kohlenmonoxidreichen Stromes, und dass
- der erste, kohlenmonoxidarme Strom als Eduktstrom in der Phosgenierung von Aminen zu Isocyanaten in der Gasphase eingesetzt wird.

Es wurde gefunden, dass die Front-End-Abtrennung des Kohlenmonoxids aus dem phosgenhaltigen Reaktionsgemisch der Synthese von Kohlenmonoxid und Chlor vor der Phosgenierung der Amine zu den entsprechenden Isocyanaten ein besonders vorteilhafter, weil sehr selektiver Trennschritt ist.

Erfindungsgemäß wird mittels eines thermischen Trennverfahrens oder eines Membrantrennverfahrens aus dem phosgenhaltigen Reaktionsgemisch der Gasphasensynthese von Kohlenmonoxid und Chlor zu Phosgen ein erster, kohlenmonoxidarmer Strom abgetrennt, der maximal 1 Gew.-% Kohlenmonoxid, bezogen auf das Gesamtgewicht des ersten, kohlenmonoxidarmen Stromes, enthält.

Bevorzugt wird in der Front-End-Abtrennung der Kohlenmonoxidgehalt auf maximal 1 Gew.-% Kohlenmonoxid, bezogen auf das Gesamtgewicht des ersten, kohlenmonoxidarmen Stromes, gesenkt.

Die Gasphasensynthese von Kohlenmonoxid und Chlor zu Phosgen wird bevorzugt mit einem stöchiometrischen Überschuss von Kohlenmonoxid zu Chlor von 0,01 bis 25 % durchgeführt.

Vorteilhaft kann ein einstufiges thermisches Trennverfahren eingesetzt werden.

Weiter bevorzugt kann ein zweistufiges thermisches Trennverfahren eingesetzt werden.

In einer weiteren bevorzugten Ausführungsform kann ein mehrstufiges thermisches Trennverfahren eingesetzt werden.

Das ein-, zwei- oder mehrstufige Trennverfahren kann aus einer einfachen Trennung durch partielle Kondensation, einer Destillation, einer Adsorption oder einer Absorption ausgewählt sein.

Das thermische Trennverfahren kann mit einem Membrantrennverfahren gekoppelt werden. In einer weiteren Ausführungsform erfolgt die Auftrennung des phosgenhaltigen Reaktionsgemisches aus der Gasphasensynthese von Kohlenmonoxid und Chlor zu Phosgen mittels eines Membrantrennverfahrens.

Vorteilhaft wird der erste, kohlenmonoxidarme Strom in flüssiger Form gewonnen und anschließend verdampft und dann in einer Gasphasenphosgenierung von Aminen eingesetzt.

Aus dieser Verfahrensführung ergeben sich weitere Vorteile:
Flüssiges Phosgen lässt sich einfach mittels einer Pumpe auf ein höheres Druckniveau bringen. So kann der phosgenhaltige Strom ohne eine direkte Kopplung der Druckniveaus der Phosgensynthese und dem Gasphasenphosgenierverfahren für die Gasphasenphosgenierung bereitgestellt werden. So ist es prinzipiell möglich die Phosgenverdampfung, die Phosgenüberhitzung oder die Gasphasenphosgenierung an sich bei einem höheren Druck als die Phosgensynthese zu betreiben.

Besonders vorteilhaft kann zur Verdampfung des phosgenhaltigen kohlenmonoxidarmen Stromes die Reaktionswärme aus der Gasphasensynthese von Kohlenmonoxid und Chlor genutzt werden. Die Phosgensynthese liefert dabei so viel Energie, dass das Phosgen gegebenenfalls nach der Verdampfung auch noch überhitzt werden kann.

Es ist jedoch auch prinzipiell möglich die gegebenenfalls fehlende Energie zur Verdampfung oder Überhitzung durch elektrische Beheizung oder Dampfbeheizung bereitzustellen.

Die wärmetechnische Kopplung der Phosgensynthese und der Verdampfung des kondensierten Phosgens kann direkt erfolgen, indem man das zu verdampfende Phosgen durch den Außenraum des Phosgenreaktors leitet. Diese Verfahrensführung hat sicherheitstechnische Vorteile gegenüber einer Kühlung des Phosgenreaktors mit dem Wärmeträger Wasser, da im Falle einer Leckage kein Wasser in den Phosgenreaktor gelangen kann. Für die wärmetechnische Kopplung der Phosgensynthese und der Phosgenverdampfung kann auch ein sekundärer Wärmeträger, wie z.B. ein Wärmeträgeröl genutzt werden.

Es ist jedoch auch möglich, mit der Abwärme der Phosgensynthese zuerst Dampf zu erzeugen und anschließend diesen zu nutzen, um das Phosgen zu verdampfen.

Bevorzugt wird der erste, durch ein ein-, zwei- oder mehrstufiges thermisches Trennverfahren und/oder ein Membrantrennverfahren gewonnene kohlenmonoxidarme Strom auf einem um mindestens 10mbar niedrigeren Druckniveau gegenüber dem ein-, zwei- oder mehrstufigen Trennverfahren und/oder Membrantrennverfahren zur Auftrennung des phosgenhaltigen Reaktionsgemisches aus der Gasphasensynthese von Kohlenmonoxid und Chlor verdampft.

Wird das Phosgen auf einem höheren Druckniveau kondensiert gegenüber dem Druckniveau, auf welches das kondensierte Phosgen anschließend entspannt wird, ist eine direkte Wärmekopplung möglich, d.h. die Kondensationswärme ist ausreichend zur erneuten Verdampfung des Phosgens, und somit eine optimale Ausnutzung möglich. Zusätzliche zur Verdampfung notwendige Energie kann aber auch durch bekannte technische Energiebereitstellung erfolgen.

Vorteilhaft kann der zweite, kohlenmonoxidreiche Strom, enthaltend mehr als 10 Gew.-% Kohlenmonoxid, bezogen auf das Gesamtgewicht des zweiten, kohlenmonoxidreichen Stromes, in die Phosgensynthese recycliert werden.

In einer bevorzugten Ausführungsform ist das thermische Trennverfahren eine partielle Kondensation und die aus der Kondensation frei werdende Wärme wird für die Verdampfung des ersten, kohlenmonoxidarmen Stromes genutzt.

Gegenstand der Erfindung ist auch die Verwendung des als Koppelprodukt in einem vorstehend beschriebenen Verfahren anfallenden Chlorwasserstoffes zum Einsatz in einer Oxychlorierung, in einem Deacon-Verfahren zur Herstellung von Chlor oder in einer Elektrolyse.

Indem erfindungsgemäß eine Front-End-Abtrennung von Kohlenmonoxid vor dem Einsatz des phosgenhaltigen Eduktstromes in der Phosgenierung durchgeführt wird, enthält auch der chlorwasserstoffhaltige Koppelstrom nur noch sehr geringe Anteile an Kohlenmonoxid, so dass er vorteilhaft in den obigen Verfahren als Eduktstrom eingesetzt werden kann.

## Patentansprüche

1. Verfahren zur Herstellung von Isocyanaten durch Phosgenierung der entsprechenden Amine in der Gasphase, **dadurch gekennzeichnet, dass**
- ein phosgenhaltiges Reaktionsgemisch aus der Gasphasensynthese von Kohlenmonoxid und Chlor zu Phosgen, die mit einem stöchiometrischen Kohlenmonoxid-Überschuss gegenüber Chlor gefahren wird,
- mittels eines thermischen Trennverfahrens und/oder eines Membrantrennverfahrens in zwei Ströme aufgeteilt wird, und zwar in einen ersten, kohlenmonoxidarmen Strom, enthaltend maximal 1 Gew.-% Kohlenmonoxid, bezogen auf das Gesamtgewicht des ersten, kohlenmonoxidarmen Stromes, sowie in
- einen zweiten, kohlenmonoxidreichen Strom, enthaltend mehr als 10 Gew.% Kohlenmonoxid, bezogen auf das Gesamtgewicht des zweiten, kohlenmonoxidreichen Stromes, und dass
- der erste, kohlenmonoxidarme Strom als Eduktstrom in der Phosgenierung von Aminen zu Isocyanaten in der Gasphase eingesetzt wird.

2. Verfahren nach Anspruch 1, **dadurch gekennzeichnet, dass** der stöchiometrische Überschuss von Kohlenmonoxid zu Chlor in der Gasphasensynthese von Phosgen 0,01 bis 25 Gew.-% beträgt.

3. Verfahren nach Anspruch 1 oder 2, **dadurch gekennzeichnet, dass** das thermische Trennverfahren ein-, zwei- oder mehrstufig durchgeführt und aus einer partiellen Kondensation, einer Destillation, einer Adsorption oder einer Absorption ausgewählt ist.

4. Verfahren nach einem der Ansprüche 1 bis 3, **dadurch gekennzeichnet, dass** der erste, kohlenmonoxidarme Strom in flüssiger Form gewonnen und anschließend verdampft wird.

5. Verfahren nach Anspruch 4, **dadurch gekennzeichnet, dass** die Reaktionswärme aus der Gasphasensynthese von Kohlenmonoxid und Chlor zu Phosgen für die Verdampfung des in flüssiger Form gewonnenen ersten kohlenmonoxidarmen Stromes genutzt wird.

6. Verfahren nach Anspruch 4 oder 5, **dadurch gekennzeichnet, dass** der erste, durch ein ein-, zwei- oder mehrstufiges thermisches Trennverfahren und/oder ein Membrantrennverfahren gewonnene kohlenmonoxidarme Strom auf einem um mindestens 10 mbar niedrigerem Druckniveau gegenüber dem ein-, zwei- oder mehrstufigen thermischen Trennverfahren und/oder Membrantrennverfahren zur Auftrennung des phosgenhaltigen Reaktionsgemisches aus der Gasphasensynthese von Kohlenmonoxid und Chlor verdampft wird.

7. Verfahren nach einem der Ansprüche 4 bis 6, **dadurch gekennzeichnet, dass** das thermische Trennverfahren eine partielle Kondensation ist und dass für die Verdampfung des ersten, kohlenmonoxidarmen Stromes die aus der Kondensation frei werdende Wärme genutzt wird.

8. Verfahren nach einem der Ansprüche 1 bis 7, **dadurch gekennzeichnet, dass** der zweite, kohlenmonoxidreiche Strom, enthaltend mehr als 10 Gew-% Kohlenmonoxid, bezogen auf das Gesamtgewicht des zweiten, kohlenmonoxidreichen Stromes, in die Phosgensynthese recycliert wird.

9. Verwendung des als Koppelprodukt in einem Verfahren nach einem der Ansprüche 1 bis 8 anfallenden Chlorwasserstoffes zum Einsatz in einer Oxichlorierung, in einem Deacon-Verfahren zur Herstellung von Chlor oder in einer Elektrolyse.

## Claims

1. A process for preparing isocyanates by phosgenating the corresponding amines in the gas phase, which comprises
- dividing a phosgene-containing reaction mixture from the gas phase synthesis of carbon monoxide and chlorine to phosgene, which is run with a stoichiometric carbon monoxide excess over chlorine,
- by means of a thermal separating process and/or of a membrane separating process into two streams, specifically into a first, low-carbon monoxide stream comprising not more than 1% by weight of carbon monoxide, based on the total weight of the first, low-carbon monoxide stream, and into
- a second, carbon monoxide-rich stream comprising more than 10% by weight of carbon monoxide, based on the total weight of the second, carbon monoxide-rich stream, and
- using the first, low-carbon monoxide stream as a reactant stream in the phosgenation of amines to isocyanates in the gas phase.

2. The process according to claim 1, wherein the stoichiometric excess of carbon monoxide to chlorine in the gas phase synthesis of phosgene is 0.01 to 25% by weight.

3. The process according to claim 1 or 2, wherein the thermal separating process is performed in one, two or more than two stages and is selected from a partial condensation, a distillation, an adsorption and an absorption.

4. The process according to any of claims 1 to 3, wherein the first, low-carbon monoxide stream is obtained in liquid form and then vaporized.

5. The process according to claim 4, wherein the heat of reaction from the gas phase synthesis of carbon monoxide and chlorine to phosgene is used for the vaporization of the first, low-carbon monoxide stream obtained in liquid form.

6. The process according to claim 4 or 5, wherein the first, low-carbon monoxide stream obtained by a one-, two- or multistage thermal separating process and/or a membrane separating process is vaporized to a pressure level at least 10 mbar lower compared to the one-, two- or multistage thermal separating process and/or membrane separating process for separation of the phosgene-containing reaction mixture from the gas phase synthesis of carbon monoxide and chlorine.

7. The process according to any of claims 4 to 6, wherein the thermal separating process is a partial condensation, and the heat released from the condensation is utilized for the vaporization of the first, low-carbon monoxide stream.

8. The process according to any of claims 1 to 7, wherein the second, carbon monoxide-rich stream comprising more than 10% by weight of carbon monoxide, based on the total weight of the second, carbon monoxide-rich stream, is recycled into the phosgene synthesis.

9. The use of the hydrogen chloride obtained as a coproduct in a process according to any of claims 1 to 8 for use in an oxychlorination, in a Deacon process for preparation of chlorine or in an electrolysis.

## Revendications

1. Procédé de fabrication d'isocyanates par phosgénation des amines correspondantes en phase gazeuse, **caractérisé en ce que**
- un mélange réactionnel contenant du phosgène issu de la synthèse en phase gazeuse de monoxyde de carbone et de chlore en phosgène, qui est réalisée avec un excès stoechiométrique de monoxyde de carbone par rapport au chlore,
- est divisé par un procédé de séparation thermique et/ou un procédé de séparation sur membrane en deux courants, à savoir en un premier courant pauvre en monoxyde de carbone, contenant au plus 1 % en poids de monoxyde de carbone, par rapport au poids total du premier courant pauvre en monoxyde de carbone, et en
- un second courant riche en monoxyde de carbone, contenant plus de 10 % en poids de monoxyde de carbone, par rapport au poids total du second courant riche en monoxyde de carbone, et **en ce que**
- le premier courant pauvre en monoxyde de carbone est utilisé en tant que courant de réactifs dans la phosgénation d'amines en isocyanates en phase gazeuse.

2. Procédé selon la revendication 1, **caractérisé en ce que** l'excès stoechiométrique de monoxyde de carbone par rapport au chlore lors de la synthèse en phase gazeuse de phosgène est de 0,01 à 25 % en poids.

3. Procédé selon la revendication 1 ou 2, **caractérisé en ce que** le procédé de séparation thermique est réalisé en une ou deux étapes ou plus et choisi parmi une condensation partielle, une distillation, une adsorption ou une absorption.

4. Procédé selon l'une quelconque des revendications 1 à 3, **caractérisé en ce que** le premier courant pauvre en monoxyde de carbone est obtenu sous forme liquide, puis vaporisé.

5. Procédé selon la revendication 4, **caractérisé en ce que** la chaleur de réaction issue de la synthèse en phase gazeuse de monoxyde de carbone et de chlore en phosgène est utilisée pour l'évaporation du premier courant pauvre en monoxyde de carbone obtenu sous forme liquide.

6. Procédé selon la revendication 4 ou 5, **caractérisé en ce que** le premier courant pauvre en monoxyde de carbone obtenu par un procédé de séparation thermique à une ou deux étapes ou plus et/ou par un procédé de séparation sur membrane est évaporé à un niveau de pression inférieur d'au moins 10 mbar par rapport au procédé de séparation thermique à une ou deux étapes ou plus et/ou au procédé de séparation sur membrane pour la séparation du mélange réactionnel contenant du phosgène issu de la synthèse en phase gazeuse de monoxyde de carbone et de chlore.

7. Procédé selon l'une quelconque des revendications 4 à 6, **caractérisé en ce que** le procédé de séparation thermique est une condensation partielle et **en ce que** la chaleur libérée par la condensation est utilisée pour l'évaporation du premier courant pauvre en monoxyde de carbone.

8. Procédé selon l'une quelconque des revendications 1 à 7, **caractérisé en ce que** le second courant riche en monoxyde de carbone, contenant plus de 10 % en poids de monoxyde de carbone, par rapport au poids total du second courant riche en monoxyde de carbone, est recyclé dans la synthèse de phosgène.

9. Utilisation du chlorure d'hydrogène formé en tant que sous-produit dans un procédé selon l'une quelconque des revendications 1 à 8 dans une oxychloration, dans un procédé de Deacon pour la fabrication de chlore ou dans une électrolyse.
